# EUROPÄISCHE PATENTSCHRIFT

(11) **EP 2 594 367 B1**
(45) Veröffentlichungstag und Bekanntmachung des Hinweises auf die Patenterteilung: **22.01.2014**
(21) Anmeldenummer: 11190411.6
(22) Anmeldetag: 23.11.2011
(51) Int. Cl.: B25B 15/02, B25B 23/142, A61B 17/88, F16D 7/04

(54) **Schraubwerkzeug mit einer Vorrichtung zur Begrenzung des übertragenen Drehmomentes**
Screw tool with a device for limiting the transferred torque
Outil de vissage doté d'un dispositif de limitation du couple transmis

(30) Priorität: 07.11.2011 CH 17822011
(43) Veröffentlichungstag der Anmeldung: 22.05.2013
(73) Patentinhaber: PB Swiss Tools GmbH, 3457 Wasen im Emmental (CH)
(72) Erfinder: Schüpbach, Peter, 3457 Wasen (CH)
(74) Vertreter: Liebetanz, Michael

(56) Entgegenhaltungen:
- EP-A1- 1 514 645
- WO-A1-2005/077603

## Beschreibung

### TECHNISCHES GEBIET

Die vorliegende Erfindung betrifft ein Schraubwerkzeug mit einer Vorrichtung zur Begrenzung des übertragenen Drehmomentes, insbesondere mit einer in der Höhlung eines Griffteiles angeordneten Vorrichtung zur Begrenzung des übertragenen Drehmomentes, bestehend aus dem Griffteil als Antriebsteil, einem in der Vorrichtung gelagerten Schaft als Abtriebsteil und zwischen dem Griffteil und dem Schaft angeordneten Kupplungselementen, die bei Überschreiten eines vorgewählten Drehmomentes die Weiterleitung des Drehmomentes vom Antriebsteil zum Abtriebsteil unterbrechen, wobei die Kupplungselemente zwei Mitnehmerbüchsen mit in eine Rastbuchse eingreifenden Verzahnungen umfassen, wobei eine Druckfeder mit einer auf die Kupplungselemente wirkenden Vorspannung in dem Griffteil angeordnet ist, wobei die Mitnehmerbüchsen gegen eine Verdrehung um die Werkzeugachse gesichert mit dem Griffteil verbunden sind, während die Rastbuchse gegen eine Verdrehung um die Werkzeugachse gesichert und bezüglich dem Schaft axial verschiebbar ist, gemäss dem Oberbegriff des Anspruch 1.

### STAND DER TECHNIK

Ein solches Schraubwerkzeug ist aus der EP 1 514 645 bekannt. Das Schraubwerkzeug verfügt über eine in der Höhlung eines Griffes angeordnete Vorrichtung zur Begrenzung des übertragenen Drehmomentes, bestehend aus dem Griff als Antriebsteil, einem in der Vorrichtung gelagerten Schaft als Abtriebsteil und zwischen dem Griff und dem Schaft angeordneten Kupplungselementen, die bei Überschreiten eines vorgewählten Drehmomentes die Weiterleitung des Drehmomentes vom Antriebsteil zum Abtriebsteil unterbrechen.

Gemäss der EP 1 514 645 besteht die Vorrichtung aus zwei Kupplungen, die aus einer ersten und einer zweiten Rastbuchse und einer ersten und zweiten Mitnehmerbüchse sowie einer Druckfeder bestehen, die mit Vorspannung zwischen den Kupplungen auf einer Zahnhülse angeordnet sind. Dabei weisen die aufeinander zugewandten Stirnseiten der Rastbuchsen und Mitnehmerbüchsen ineinander greifende Verzahnungen auf, sodass die Mitnehmerbüchsen formschlüssig und drehfest mit dem Griff verbunden sind und drehbar auf der Zahnhülse gelagert. Dabei greifen die Rastbuchsen ferner mit einer Innenverzahnung in eine Formgleiche Aussenverzahnung der Zahnhülse ein und sind auf dieser drehfest und axial verschiebbar.

Der Vorteil dieser Vorrichtung gemäss dem Stand der Technik besteht insbesondere darin, dass die wesentlichen Teile aus Kunststoff im Spritzgiessverfahren herstellbar sind und trotzdem höhere Drehmomente übertragen werden können. Die Begrenzung des Drehmomentes werden gemäss dem Stand der Technik an zwei Stellen in die Drehmomentbegrenzungsmechanik übertragen, welche durch Kupplungen vorgespannt sind, die durch eine zwischen diesen Kupplungen angeordnete Druckfeder mit einer Vorspannung beauftragt werden.

### DARSTELLUNG DER ERFINDUNG

Ausgehend von diesem Stand der Technik geht die Erfindung von der Aufgabe aus, eine einfacher herzustellende, weniger Teile umfassende Vorrichtung anzugeben, mit der die Einstellung des Auslösemomentes über eine längere Zeitdauer konstant bleibt.

Auch soll die einfachere Vorrichtung zur Begrenzung des Drehmomentes mindestens eine gleich grosse Drehmomentbegrenzung wie der Stand der Technik leisten.

Ein weiteres Ziel ist es, eine einfachere Einstellbarkeit und Nachstellbarkeit des Auslösemomentes vorzugeben, also als eine weitere Aufgabe der vorliegenden Erfindung einen Einstellmechanismus anzugeben, mit dem der vorbestimmte Wert der Drehmomentbegrenzung in einfacher weise nachjustiert werden kann.

Diese Ziele werden mit einer Vorrichtung mit den Merkmalen des Anspruchs 1 gelöst, insbesondere verfügt ein Werkzeug mit den Merkmalen nach dem Oberbegriff des Anspruchs 1 über eine einzige Rastbuchse, die zwei stirnseitige Eingriffsflächen aufweist, die mit ihren Verzahnungen in jeweilige Eingriffsflächen der beiden gegenüberliegenden Mitnehmerbüchsen eingreifen, wobei die Rastbuchse eine Innenkontur und der Schaft eine komplementäre Aussenkontur aufweist, mit der eine Verdrehung der Rastbuchse gegenüber dem Schaft um die Werkzeugachse verhindert wird, wobei die Mitnehmerbüchsen eine Aussenkontur und das Griffteil eine komplementäre Innenkontur aufweisen, mit der eine Verdrehung der Mitnehmerbüchsen gegenüber dem Griffteil verhindert wird und mit denen die Mitnehmerbüchsen gegenüber dem Griffteil axial verschiebbar sind, und wobei schliesslich die Druckfeder zwischen den Kupplungselementen und einer Gegenfläche des Griffteils angeordnet ist.

Weitere Ausführungsformen sind in den abhängigen Ansprüchen angegeben.

Das Schraubwerkzeug mit einer Drehmomentbegrenzung kann insbesondere vorteilhaft auch im Medizinalbereich eingesetzt werden, da dort eine sichere einmalige Anwendung des noch nie gebrauchten Werkzeugs ohne Reinigung und Sterilisation möglich ist, woraus sich eine konstante Verfügbarkeit ergibt.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: zeigt eine teilweise geöffnete perspektivische Ansicht des Schraubwerkzeuges gemäss einem Ausführungsbeispiel der Erfindung.
- Fig. 2: zeigt eine perspektivische Explosionsansicht der einzelnen Elemente der das Drehmoment begrenzenden Vorrichtung des Schraubwerkzeuges gemäss Fig. 1, und
- Fig. 3: zeigt eine teilweise geschnittene Seitenansicht des Schraubwerkzeuges nach Fig. 1.

### BESCHREIBUNG BEVORZUGTER AUSFÜHRUNGSFORMEN

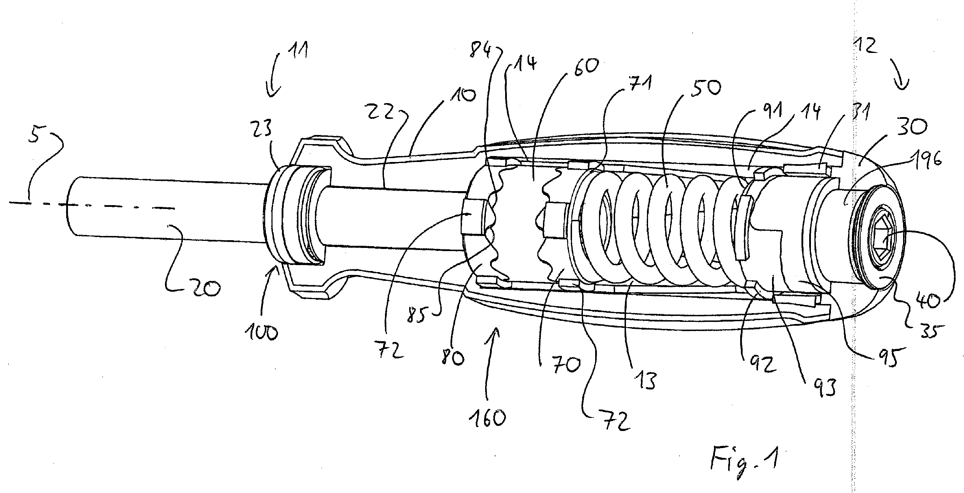

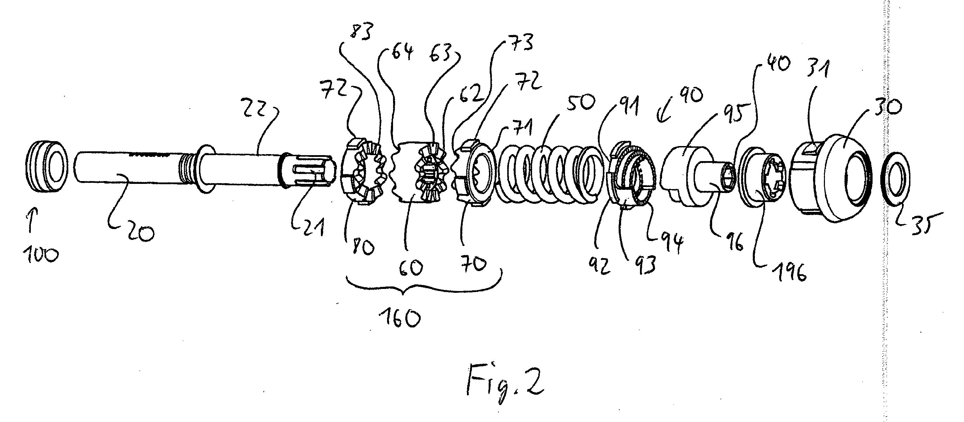

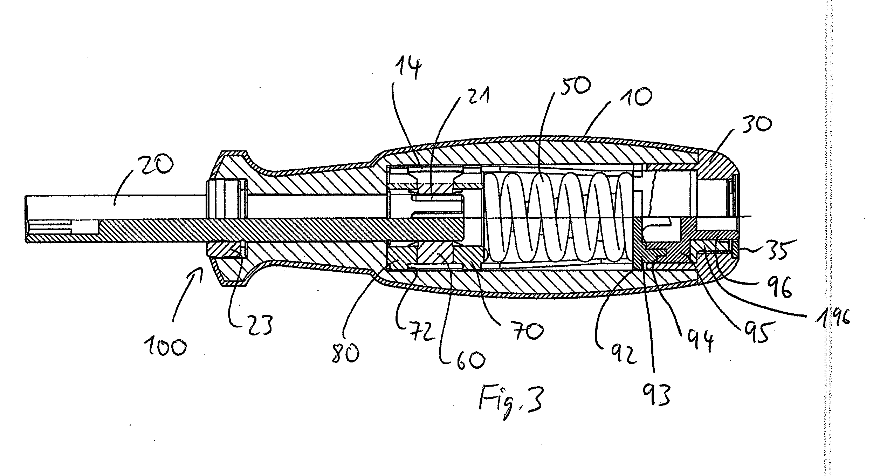

Die Fig. 1 zeigt eine teilweise geöffnete perspektivische Ansicht eines Schraubwerkzeuges mit einer Vorrichtung zur Begrenzung des übertragenen Drehmomentes gemäss einem Ausfiihrungsbeispiels gemäss der Erfindung. Teilweise ist die Erfindung in der eine perspektivische Explosionsansicht der einzelnen Elemente der das Drehmoment begrenzenden Vorrichtung des Schraubwerkzeuges zeigenden Fig. 2 oder der eine teilweise geschnittene Seitenansicht des Schraubwerkzeuges zeigenden Fig. 3 besser zu erkennen, so dass auf diese hier auch direkt Bezug genommen wird.

Das Schraubwerkzeug hat eine umfassende, innen hohle Griffhülse 10, die sich entlang der Drehachse beziehungsweise Hauptachse 5 des Werkzeugs erstreckt. Die Griffhülse 10 weist an ihren beiden Enden, dem werkzeugseitigen Ende 11 und dem einstellungsseitigen Ende 12, jeweils eine Öffnung auf. An dem werkzeugseitigen Ende 11 besteht eine Arbeitsöffnung zur Aufnahme eines Schaftes 20. Auf der gegenüberliegenden Einstellseite ist die Öffnung durch eine Kappe 30 verschlossen, die in die Griffhülse 10 zu einem Formschluss mit einer Raste 31 einklipsbar ist. Die Verbindung zwischen Kappe 30 und Griffhülse 10 kann aber auch durch ein Gewinde oder beispielsweise eine Schallschweissung der beiden Elemente 10 und 30 realisiert sein.

Im mittleren hohlen Abschnitt 13 der Griffhülse 10 ist eine Druckfeder 50 angeordnet. Diese Druckfeder 50 stützt sich an ihren beiden Enden einerseits auf der schaftfernen Ringfläche 71 der ersten Mitnehmerbüchse 70 und andererseits auf der schaftseitigen, geschlossenen Fläche 91 des Einstellmechanismus 90 ab. Prinzipiell wäre es möglich, was aber nicht notwendig ist, einen Führungsstab im durch die Druckfeder 50 aufgespannten Hohlraum 13 entlang der Hauptachse 5 vorzusehen, der natürlich nicht die ganze Länge der Feder 50 einnehmen dürfte, da diese im Betrieb über eine gewisse axiale Länge zusammengedrückt wird. Solch ein Führungsstab könnte beispielsweise einstückig an der schaftseitigen, geschlossenen Fläche 91 des Einstellmechanismus 90 angespritzt sein. Oder der Schaft 20 des Werkzeuges selber könnte sich durch die zweite Mitnehmerbüchse 80 und die erste Mitnehmerbüchse 70 hindurch- und teilweise in den Innenraum 13 der Feder 50 hineinerstrecken,

Wesentliches Element der Vorrichtung zur Begrenzung des übertragenen Drehmomentes umfasst die beiden Mitnehmerbüchsen 70 und 80 und das dazwischen vorgesehene Kupplungsteil in Gestalt einer Rastbuchse 60. Diese drei Elemente bilden ein zusammengehöriges Paket 160 entlang der Hauptachse 5.

Die Rastbuchse 60 verfügt über einen inneren Hohlraum 61 mit einer vorbestimmten Innenkontur 62, in die die komplementäre Aussenkontur 21 des Schaftes 20 eingreift. Die Aussenkontur 21 wird durch sechs längs verlaufende Nuten gebildet, der sechs in den Innenraum der Rastbuchse 60 überstehende Grate oder Kämme gegenüberstehen. Natürlich können auch ein, zwei oder eine andere Anzahl von Nuten vorgesehen sein mit einer entsprechenden Anzahl von Graten. Es können auch die Funktionen von Grat und Nuten vertauscht sein und die Aussenkontur über den zylindrischen Schaft hinausgehend Grate aufweisen. Es wird somit zwischen der Rastbuchse 60 und dem Schaft 20 ein gegen Drehung um die Achse 5 gesichertes System geschaffen, wobei die Rastbuchse 60 sich aber in Längsrichtung der Hauptachse 5 des Schaftes 20 auf dessen Aussenkontur 21 bewegen kann. Die Aussenkontur 21 ist daher auf einem längeren achsialen Abschnitt des Schaftes 20 vorgesehen, so dass sich die Rastbuchse 60 entlang von diesem Bereich durch entsprechendes Spiel bewegen kann.

Die weitere frontseitige Aussenkontur 22 des Schaftes 20 kann insbesondere rotationssymmetrisch ausgestaltet sein, so dass die auf der Innenseite der Griffhülse 10 vorgesehene in eine entsprechende Ausnehmung der Griffhülse 10 eingesetzte Lagerung 23 den Schaft 20 in einer gegenüber der Griffhülse 10 drehbaren Weise durchtreten lässt.

Die beiden Mitnehmerbüchsen 70 und 80 auf den beiden Seiten der Rastbuchse 60 weisen jeweils eine Aussenkontur 72 auf, die mit einer komplementären Innenkontur 14 der Griffhülse 10 in einem drehsichereren Eingriff steht. Vorteilhafterweise umfasst die Aussenkontur 72 eine Vielzahl von in diesem Ausführungsbeispiel vier Grate, die in einem entlang dem Umfang gemessenen Winkel von 90 Grad zueinander angeordnet sind. Dementsprechend sind vier längs verlaufende Nuten in der Griffhülse vorgesehen, in denen die entsprechenden Grate 72 der Mitnehmerbüchsen 70 und 80 entlang gleiten können.

Demgemäss sind die Innendurchmesser der Mitnehmerbüchsen 70 und 80 ausreichend bemessen, so dass sich mindestens die Aussenkontur 21 des Schaftes 20 durch diese hindurcherstrecken kann.

Die Rastbuchse 60 weist an ihren longitudinalen Enden über Eingriffsflächen 63 beziehungsweise 64 auf, die mit entsprechend auf diese ausgerichtete Eingriffsflächen 73 und 83 auf den ihnen zugewandten Flächen jeweils auf den beiden Mitnehmerbüchsen 70 beziehungsweise 80 vorgesehen sind.

Diese beiden Flächenpaare 63-73 und 64-83 von Rastbuchse 60 und Mitnehmerbüchse 70 beziehungsweise von Rastbuchse 60 und Mitnehmerbüchse 80 sind vorteilhafterweise zueinander gleich ausgestaltet.

Die Flächenpaare weisen jeweils Abfolgen von Rampenteilen auf, was jeweils eine in Umfangsrichtung des Werkzeuges ausgerichtete Rampe umfasst, die in Umfangsrichtung auf dem - in Draufsicht - Kreisring ansteigt. Dabei sind die Steigungen entgegengesetzt ausgerichtet. So besteht eine Rampe 84 mit geringer Steigerung, der sich eine Rampe 85 mit starkem Abfall anschliesst. Diese Abfolge von Rampenteilen 84-85 wiederholt sich auf dem Umfang bei dem Ausführungsbeispiel zehnmal, so dass jedes Rampenteilpaar 84-85 einen Winkel von 36 Grad überstreicht. Natürlich können auch grössere Winkel wie vier Rampenteilpaare 84-85 mit je 90 Grad oder kleinere vorgesehen sein.

Wird nun die Griffhülse 10 gedreht, so drehen sich die beiden Mitnehmerbüchsen 70 und 80 auf Grund ihres radialen Formschlusses mit der Innenkontur der Griffhülse 10 mit. Durch den Reibschluss zwischen den besagten Flächenpaaren der Rampen dreht auch die Rastbuchse 60 in gleicher Richtung mit. Diese Rastbuchse 60 treibt über den in radialer Richtung bestehenden Formschluss mit dem Schaft 20 diesen an, so dass sich schlussendlich das an dessen Spitze befindliche Werkzeugbit dreht. Diese Drehung des Werkzeugbits erfolgt gegen eine Kraft, die über die radialen Abstände ein Drehmoment im Werkzeug erzeugt. Dieses Drehmoment wird über den Schaft 20 auf die Rastbuchse 60 übertragen und führt bei einer steigenden Kraft zu einer Verschiebung der Rampenteile 84 und 85 gegeneinander, so dass sich die Rastbuchse 60 einfach gegenüber der ersten Mitnehmerbüchse 70 dreht und sich axial von dieser entfernt. Das gleiche gilt für das System Mitnehmerbüchse 80 zu Rastbuchse 60, welches sich bei gleichem longitudinalen Verschiebeweg entlang der Achse 5 doppelt so weit von der Spitze des Werkzeugs entfernt. Dieses Verschieben geschieht gegen die Kraft der Druckfeder 50, die durch das Verschieben zusammengedrückt wird. Damit erhöht sich die auf die Eingriffsflächen 73 beziehungsweise 83 ausgeübte Kraft auf die Mitnehmerbüchsen 70 und 80, respektive.

Durch die somit beidseitig auf den Rampen 84, 85 auflaufende Rastbuchse 60 werden die beiden ineinandergreifenden Verzahnungen entlang der Achse 5 näher zueinander gebracht und die Rastbuchse 60 kann kürzer ausgestaltet werden. Dadurch kann ein Verschleppen der Verdrehung der Rastbuchse 60 durch eine Verwindung derselben sicher vermieden werden, denn es ist in Längsrichtung der Rastbuchse 60 kein entsprechendes Material vorhanden, weil die Rastbuchse 60 kurz ist. Auch kann der effektive Federweg verdoppelt werden, ohne eine komplexe Innenmechanik wie beim Stand der Technik vorauszusetzen.

Es ist fertigungstechnisch von Vorteil, dass die Innenkontur der Griffhülse 10 sowohl für die Mitnehmerhülsen 70 und 80 als auch für den Einstellmechanismus 90 verwendet werden kann. Auf dem Einstellmechanismus 90 sind entsprechende eine Verdrehsicherung 92 bildende Grate vorgesehen. Es ist dann abgesehen von dem Einklipsmechanismus oder den entsprechenden Flächen zur Ausführung einer Verschweissung keine weitergehende Verzahnung zum Abschluss des Werkzeugs notwendig.

Durch das Verschieben des Druckelementes hinter das Zahnpaket 160 können die der Verzahnung gegenüberliegenden Auflageflächen der Mitnehmerhülsen 70 und 80 gleich ausgestaltet werden. Eine solche identische Ausformung vermindert die Fertigungskosten.

Ferner ermöglicht die identische Form der Verzahnung auch den Einbau der Elemente für eine rechtsdrehende Momentbegrenzung beziehungsweise für eine linksdrehende Momentbegrenzung. Das Paket 160 aus Mitnehmerhülsen 70 und 80 und dazwischen angeordneter Rastbuchse 60 braucht nur um 180 Grad gegenüber einer senkrecht zur Längsachse des Werkzeuges stehenden Achse verdreht zu werden, was faktisch zu einem Wechsel oder Austausch zwischen Rampe 84 mit geringer Steigung und Rampe 85 mit starkem Abfall fuhrt.

Der Einstellmechanismus 90 besteht aus einem Spritzgussteil 93 mit drei um jeweils 120 Grad zueinander verdrehten Rastkurven 94, die konzentrisch zueinander angeordnet sind. Prinzipiell wäre auch eine einzige Rastkurve 94 ausreichend. Die Verwendung von mehreren Rastkurven 94 gestattet eine genauere Einstellung über eine längere Zeit, da weniger Verschleiss auf jede Rastkurve 94 übertragen wird. Die ansteigend wellige Form der Rastkurven 94 arbeitet mit einer Einstellrampe auf dem Gegenstück 95 zusammen. Dies ermöglicht einen Einstellmechanismus in nur zwei Teilen. Jede Rastkurve 94 überdeckt in einer 360 Grad Kurve einen bis auf die Rastnasen axial ansteigenden Bereich, der dann wieder steil auf das Basisniveau abfällt. Auf dem Gegenstück 95 sind hier drei komplementäre Rastkurven vorgesehen.

Das Gegenstück endet in einem zylindrischen Stutzen 96, der von einer Korrekturschraube 196 umgeben ist, deren Flansch auf dem Absatz des zylindrischen Stutzens 96 endet. Diese beiden Elemente werden durch die hohle Kappe 30 abgedeckt, wobei die Frontfläche des zylindrischen Stutzens 96 durch die Kappe 30 hindurchsteht, während die kreisringförmige Frontfläche der Korrekturschraube 196 von einem flachen Ring 35 abgedeckt wird.

Die Korrekturschraube 196 ist über ein in der Kappe vorgesehenes komplementäres Gewinde axial gegenüber der Kappe 30 verschiebbar. Dafür ist der flache Ring 35 abzunehmen, um mit einem Werkzeug in die in der Fig. 2 erkennbaren Ausnehmungen in der Frontpartie der Korrekturschraube 196 einzugreifen. Der Innensechskant 40 ist dagegen für die Verdrehung des Gegenstückes 95 des Einstellmechanismus 90 vorgesehen, da dieser in dem mit dem Gegenstück 95 verbundenen Stutzen 96 angeordnet ist.

Eine Korrekturschraube 196 ist zwischen Einstellmechanismus 90 und der Kappe 30 vorgesehen. Diese Korrekturschraube 196 ermöglicht eine Korrektur des Einstellwertes der Drehmomentbegrenzung, also der Auslösung des Durchrutschens der Rastbuchse 60 gegenüber den Mitnehmerhülsen 70 und 80. Dies kann zur Rekalibrierung zu gegebener Zeit vorgenommen werden. Funktional wird das Gegenstück 95 mit dem Rastkurventeil 93 zueinander verdreht, so dass die schaftseitige geschlossene Fläche 91 des Rastkurventeils 93 axial in Richtung des werkzeugseitigen Endes 11 verschoben wird, was zu einer erhöhten Vorspannung der Feder 50 führt. Durch das Verdrehen der Korrekturschraube 196 in oder gegen den Uhrzeigersinn wird der Einstellmechanismus bestehend aus dem auf der werkzeugseitigen Schulter aufliegenden Gegenstück 95 und dem Rastkurventeil 93 axial gegen die Griffkappe 30 und damit gegenüber dem Griffteil 10 verschoben. Diese Verschiebung überlagert die axiale Verschiebung, hervorgerufen durch die Verdrehung des Gegenstücks 95 zum Rastkurventeil 93 und kann dadurch zur Kompensation eines eventuellen Kraftabfalls der Federvorspannung genutzt werden.

Da die Lagerung des Schaftes 20 des Bithalters axial und radial durch die Elemente von der Lagerung des Zahnpakets 160 durch die Längsverzahnung in der Griffhülse 10 entkoppelt ist und die Rastbuchse 60 radial beweglich auf dem Schaft 20 gelagert ist, kann dadurch eine gegenseitige Anpassung an die Zahnform stattfinden und damit ein eventueller radialer Versatz kompensiert werden Durch einseitiges Anspritzen der Kunststoffteile oder ungleichmässiges Erkalten können nämlich leichte Symmetrieabweichungen entstehen. Falls diese nicht durch entsprechende Massnahmen korrigiert werden, wäre eine periodisch auftretende Drehmomentschwankung möglich.

Alle Elemente, üblicherweise mit Ausnahme des Schaftes 20 und der Druckfeder 50 sind aus Kunststoff hergestellt; insbesondere als Spritzgussteile. Prinzipiell ist auch der Einsatz von anderen Werkstoffen für diese Elemente möglich; die Vorteile des geringen Gewichts und der einfachen Herstellung sind bei einer solchen Vorgehensweise einfach am grössten, ohne Kompromisse bei der Qualität eingehen zu müssen, da die geringen Längsdimensionen keine Verwindung zulassen. Schaft 20 und Druckfeder 50 sind üblicherweise aus Metall.

**BEZUGSZEICHENLISTE**

| | | | |
|---|---|---|---|
| 5 | Hauptachse | 64 | Eingriffsfläche |
| 10 | Griffhülse | 70 | Erste Mitnehmerbüchse |
| 11 | werkzeugseitiges Ende | 71 | schaftfernen Ringfläche |
| 12 | einstellseitiges Ende | 72 | Aussenkontur der Hülsen |
| 13 | mittlerer hohler Abschnitt | 73 | Eingriffsfläche |
| 14 | Innenkontur der Hülse | 80 | Zweite Mitnehmerbüchse |
| 20 | Schaft | 83 | Eingriffsfläche |
| 21 | Aussenkontur des Schaftes | 84 | Rampe mit geringer Steigung |
| 22 | frontseitige Aussenkontur | 85 | Rampe mit starkem Abfall |
| 23 | Lagerung des Schaftes | 90 | Einstellmechanismus |
| 30 | Kappe | 91 | schaftseitigen, geschlossenen Fläche |
| 31 | Raste | | |
| 35 | flacher Ring | 92 | Verdrehsicherung |
| 40 | Innensechskant | 93 | Rastkurventeil |
| 50 | Druckfeder | 94 | Rastkurve |
| 60 | Rastbuchse | 95 | Gegenstück |
| 61 | innerer Hohlraum der Rastbuchse | 96 | Stutzen |
| | | 100 | Lager |
| 62 | Innenkontur der Rastbuchse | 160 | Paket |
| 63 | Eingriffsfläche | 196 | Korrekturschraube |

## Patentansprüche

1. Schraubwerkzeug mit einer in der Höhlung eines Griffteiles (10) angeordneten Vorrichtung (50; 160) zur Begrenzung des übertragenen Drehmomentes, bestehend aus dem Griffteil (10) als Antriebsteil, einem in der Vorrichtung gelagerten Schaft (20) als Abtriebsteil und zwischen dem Griffteil (10) und dem Schaft (20) angeordneten Kupplungselementen (160; 60, 70, 80), die bei Überschreiten eines vorgewählten Drehmomentes die Weiterleitung des Drehmomentes vom Antriebsteil zum Abtriebsteil unterbrechen, wobei die Kupplungselemente zwei Mitnehmerbüchsen (70, 80) mit in eine Rastbuchse (60) eingreifenden Verzahnungen (84, 85) umfassen, wobei eine Druckfeder (50) mit einer auf die Kupplungselemente (160; 60, 70, 80) wirkenden Vorspannung in dem Griffteil (10) angeordnet ist, wobei die Mitnehmerbüchsen (70, 80) gegen eine Verdrehung um die Werkzeugachse (5) gesichert mit dem Griffteil (10) verbunden (72) sind, während die Rastbuchse (60) gegen eine Verdrehung um die Werkzeugachse (5) gesichert und bezüglich dem Schaft (20) axial verschiebbar ist, **dadurch gekennzeichnet, dass** die einzige Rastbuchse (60) zwei stirnseitige Eingriffsflächen (63, 64) aufweist, die mit ihren Verzahnungen (84, 85) in jeweilige Eingriffsflächen (73, 84) der beiden gegenüberliegenden Mitnehmerbüchsen (70, 80) eingreifen, dass die Rastbuchse (60) eine Innenkontur (62) und der Schaft (20) eine komplementäre Aussenkontur (21) aufweist, mit der eine Verdrehung der Rastbuchse (60) gegenüber dem Schaft (20) um die Werkzeugachse (5) verhindert wird, dass die Mitnehmerbüchsen (70, 80) eine Aussenkontur (72) und das Griffteil (10) eine komplementäre Innenkontur (14) aufweisen, mit der eine Verdrehung der Mitnehmerbüchsen (70, 80) gegenüber dem Griffteil (10) verhindert wird und mit denen die Mitnehmerbüchsen (70, 80) gegenüber dem Griffteil (10) axial verschiebbar sind, und dass die Druckfeder (50) zwischen den Kupplungselementen (60, 70, 80) und einer Gegenfläche (91) des Griffteils (10) angeordnet ist.

2. Schraubwerkzeug nach Anspruch 1, **dadurch gekennzeichnet, dass** die Kupplungselemente (160; 60, 70, 80) auf der werkzeugnahen Seite des Griffteils (10) angeordnet sind, und dass sich die Druckfeder (50) einerseits auf einer Stirnfläche (71) einer Mitnehmerbüchse (80) abstützt, und dass sie sich andererseits auf einer weiteren Stirnfläche (91) werkzeugfernen Seite des Griffteils (10) abstützt.

3. Schraubwerkzeug nach Anspruch 2, **dadurch gekennzeichnet, dass** die weitere Stirnfläche (91) eine in ihrer axialen (5) Längsposition einstellbare Fläche eines Vorspannungs-Einstellmechanismus (90) ist.

4. Schraubwerkzeug nach Anspruch 3, **dadurch gekennzeichnet, dass** der Vorspannungs-Einstellmechanismus (90) aus einem der Druckfeder (50) zugewandten Rastkurventeil (93) mit einer Rastkurve (94) und einem eine komplementäre Rastkurve aufweisenden Gegenstück (95) besteht, wobei das Gegenstück über eine Einstellmechanik verriegelbar drehbar ist.

5. Schraubwerkzeug nach Anspruch 4, **dadurch gekennzeichnet, dass** die Rastkurve (94) des Rastkurventeils (93) aus zwei oder mehr konzentrisch angeordneten, in einem regelmässigen Abstand vorgegebenen Rastkurven (94) besteht.

6. Schraubwerkzeug nach Anspruch 4 oder Anspruch 5, **dadurch gekennzeichnet, dass** der Vorspannungs-Einstellmechanismus (90) über einen in die Richtung des werkzeugfernen Seite des Griffteils (10) ragenden Betätigungsmechanismus (40), insbesondere über einen Innenmehrkant, verfügt.

7. Schraubwerkzeug nach Anspruch 6, **dadurch gekennzeichnet, dass** der Betätigungsmechanismus (40) in einem mit dem Gegenstück (95) verbundenen Stutzen (96) vorgesehen ist, der von einem Korrekturmechanismus (196) umgeben ist, der in einem Gewindeeingriff mit dem Griffteil (10) steht, um das Gegenstück (95) durch Drehung des Korrekturmechanismus (196) axial zu bewegen.

8. Schraubwerkzeug nach Anspruch 7, **dadurch gekennzeichnet, dass** Korrekturmechanismus (196) über eine werkzeugferne Betätigungsfläche verfügt, die optional durch eine für die Einstellung abnehmbare Schutzkappe (35) abgedeckt ist.

9. Schraubwerkzeug nach einem der Ansprüche 1 bis 8, **dadurch gekennzeichnet, dass** die zwei Mitnehmerbüchsen (70, 80) der Kupplungselemente (60, 70, 80) über Rampenteile (84, 85) verfügen und dass die Rampenteile (84, 85) der Kupplungselemente (60, 70, 80) unterschiedliche Steigungen aufweisen, wobei die Steigungsabfolge der Rampenteile der ersten Mitnehmerbüchse (70) gegenüber der zugeordneten Rastbuchsenfläche im genau anderen Drehsinn angeordnet sind gegenüber der Steigungsabfolge der Rampenteile der zweiten Mitnehmerbüchse (80) gegenüber der zugeordneten Rastbuchsenfläche, so dass durch eine Drehung der Kupplungselemente (60, 70, 80) als ein Paket (160) ein links- oder rechtshändig begrenzendes Schraubwerkzeug angegeben wird.

## Claims

1. Screwing tool with a device (50; 160) for limiting the transmittable torque, the device being arranged in the cavity of a handle piece (10) consisting of the handle piece (10) as a driving part, a shaft (20) that is mounted in the device as an output part and coupling elements (160; 60, 70, 80) that are arranged between the handle piece (10) and the shaft (20), the coupling elements interrupting the transmission of the torque from the driving part to the output part, when a pre-selected torque is exceeded, wherein the coupling elements comprise two driving bushes (70, 80) with cogging (84, 85) that engage with a detent bush (60), wherein a pressure spring (50) is arranged in the handle piece (10), whose pre-load is acting on the coupling elements (160; 60, 70, 80), wherein the driving bushes (70, 80) are securely connected to the handle piece (10) in order to prevent a rotation around the tool axle (5), the detent bush (60) being secured against a rotation around the tool axle (5) and being axially movable with respect to the shaft (20), **characterized in that** the sole detent bush (60) comprises two end-side engagement surfaces (63, 64) that engage with their cogging (84, 85) in respective engagement surfaces (73, 84) of the opposing driving bushes (70, 80), **in that** the detent bush (60) comprises an inner contour (62) and the shaft (20) comprises a complementary outer contour (21), with which a rotation around the tool axle (5) of the detent bush (60) with respect to the shaft (20) is prevented, **in that** the driving bushes (70, 80) comprise an outer contour (72) and the handle piece (10) comprises a complementary inner contour (14), with which a rotation of the driving bushes (70, 80) with respect to the handle piece (10) is prevented and with which the driving bushes (70, 80) are axially movable with respect to the handle piece (10), and **in that** the pressure spring (50) is arranged between the coupling elements (60, 70, 80) and a counter surface (91) of the handle piece (10).

2. Screwing tool according to claim 1, **characterized in that** the coupling elements (160; 60, 70, 80) are arranged in the handle piece (10) on a side proximal to the tool, and **in that** the pressure spring (50) on the one hand rests on an end surface (71) of a driving bush (80), and **in that** on the other hand it rests on a further end surface (91) of the handle piece (10) on a side distal to the tool.

3. Screwing tool according to claim 2, **characterized in that** the further end surface (91) is a surface of a pre-tensioning-adjustment mechanism (90) that is adjustable in its axial (5) longitudinal position.

4. Screwing tool according to claim 3, **characterized in that** the pre-tensioning-adjustment mechanism (90) consists of detent curve part (93) with a detent curve (94) facing the pressure spring (50) and a counter piece (95) that comprises a complementary detent curve, wherein the counter piece is lockable rotatable by an adjustment mechanism.

5. Screwing tool according to claim 4, **characterized in that** the detent curve (94) of the detent curve part (93) consists of two or more detent curves (94) that are arranged concentrically at a defined regular distance.

6. Screwing tool according to claim 4 or claim 5, **characterized in that** the pre-tensioning-adjustment mechanism (90) provides an actuation mechanism (40), particularly a polygonal socket that exceeds the handle piece (10) in the direction of the side that is distal to the tool.

7. Screwing tool according to claim 6, **characterized in that** the actuation mechanism (40) is provided with a stub (96) that is connected with the counter piece (95), the stub being surrounded by a correction mechanism (196) that is in a threaded engagement with the handle piece (10) in order to axially move the counter piece (95) by rotating the correction mechanism (196).

8. Screwing tool according to claim 7, **characterized in that** the correction mechanism (196) is provided with a actuation surface distal to the tool that is optionally covered by a protection cap (35) that can be removed for the adjustment.

9. Screwing tool according to one of claims 1 to 8, **characterized in that** two driving bushes (70, 80) of the coupling elements (60, 70, 80) are provided with ramp parts (84, 85) and **in that** the ramp parts (84, 85) of the coupling elements (60, 70, 80) have different inclinations, wherein the inclination sequence of the ramp parts of the first driving bush (70) is arranged in the exact opposite direction of rotation, opposing the inclination sequence of the ramp parts of the second driving bush (80) opposing the corresponding detent bush surface, so as to provide a left or right hand limiting screwing tool by the rotation of the coupling elements (60, 70, 80) as a package (160).

## Revendications

1. Outil de vissage avec un appareil (50 ; 160) pour limiter le couple transmissible, l'appareil étant arrangé dans la cavité d'une pièce de poignée (10) consistant de la pièce de poignée comme partie d'entraînement, un arbre (20) qui est monté dans l'appareil comme partie de sortie et des éléments d'accouplement (160 ; 60, 70, 80) qui sont arrangés entre la pièce de poignée (10) et l'arbre (20), les éléments d'accouplement interrompant la transmission du couple de la partie d'entraînement à la partie de sortie, si un couple présélectionné est dépassé, où les éléments d'accouplement comprennent deux douilles d'entraînement (70, 80) avec dentures (84, 85) qui s'engage avec une douille crantage (60), où un ressort de pression (50) est arrangé dans la pièce de poignée (10), la prétention duquel agit sur les éléments d'accouplement (160 ; 60, 70, 80), où les douilles d'entraînement (70, 80) sont connectés en sécurité à la pièce de poignée (10) afin d'éviter une rotation autour de l'axe (5) de l'outil, la douille crantage (60) étant connectée en sécurité contre une rotation autour de l'axe (5) de l'outil et étant mobil par rapport à l'arbre, **caractérisé en ce que** la seule douille crantage (60) comprend deux surfaces d'engagement côté extrémité (63, 64) qui s'engagent avec leur dentures (84, 85) dans des surfaces d'engagement respective (73, 84) des douilles d'entraînement opposées (70, 80), **en ce que** la douille crantage (60) comprend un contour intérieur (62) et l'arbre (20) comprend un contour extérieur complémentaire (21), avec lequel une rotation autour de l'axe de l'outil (5) de la douille crantage (60) par rapport à l'arbre (20) est empêché, **en ce que** les douilles d'entraînement (70, 80) comprennent un contour extérieur (72) et la pièce de poignée (10) comprend un contour intérieur complémentaire (14), avec lequel une rotation des douilles d'entraînement (70, 80) par rapport à la pièce de poignée (10) est empêché et avec lequel les douilles d'entraînement (70, 80) sont mobiles axialement par rapport à la pièce de poignée (10), et **en ce que** le ressort de pression (50) est arrangé entre les éléments d'accouplement (60, 70, 80) et une contre-surface (91) de la pièce de poignée (10).

2. Outil de vissage selon la revendication 1, **caractérisé en ce que** les éléments d'accouplement (160 ; 60, 70, 80) sont arrangés dans la pièce de poignée (10) sur un côté proximal de l'outil, et **en ce que** le ressort de pression (50) d'un côté est soutenu par une surface d'extrémité (71) d'une douille d'entraînement (80), et **en ce que** de l'autre côté il est soutenu par une autre surface d'extrémité (91) de la pièce poignée (10) d'un côté distal à l'outil.

3. Outil de vissage selon la revendication 2, **caractérisé en ce que** l'autre surface d'extrémité (91) est une surface d'un mécanisme de réglage de précontrainte (90) qui est réglable dans sa position axiale (5) longitudinale.

4. Outil de vissage selon la revendication 3, **caractérisé en ce que** le mécanisme de réglage de précontrainte (90) est composé d'une pièce de came d'encliquetage (93) avec une came d'encliquetage (94) qui fait face au ressort de pression (50) et une pièce contrepartie (95) qui comprend une came d'encliquetage complémentaire, où la pièce contrepartie est bloquée en rotation par un mécanisme de réglage.

5. Outil de vissage selon la revendication 4, **caractérisé en ce que** la came d'encliquetage (94) de la pièce de came d'encliquetage (93) est composé de deux ou plus cames d'encliquetage (94) qui sont arrangées concentrique à une distance régulière définie.

6. Outil de vissage selon la revendication 4 ou la revendication 5, **caractérisé en ce que** le mécanisme de réglage de précontrainte (90) fournit un mécanisme d'actionnement (40), particulièrement un polygone interne qui dépasse la pièce de poignée (10) dans la direction du côté qui est distal par rapport à l'outil.

7. Outil de vissage selon la revendication 6, **caractérisé en ce que** le mécanisme d'actionnement (40) est pourvue d'un raccord (96) qui est relié à la pièce contrepartie (95), le raccord étant entouré par un mécanisme de correction (196) qui est dans un engagement fileté avec la pièce poignée (10), afin de déplacer axialement la pièce contrepartie (95) par rotation du mécanisme de correction (196).

8. Outil de vissage selon la revendication 7, **caractérisé en ce que** le mécanisme de correction (169) est pourvue d'une surface d'actionnement distale par rapport à l'outil, qui est optionnellement couvert par un couvercle de protection (35) qui peut être enlevé pour le réglage.

9. Outil de vissage selon l'une quelconque des revendications 1 à 8, **caractérisé en ce que** les deux douilles d'entraînement (70, 80) des éléments d'accouplement (60, 70, 80) sont pourvue avec des parties de rampe (84, 85) et **en ce que** les parties de rampe (84, 85) des éléments d'accouplement (60, 70, 80) ont des inclinations différentes, où la séquence d'inclination des parties de rampe de la première douille d'entraînement (70) est arrangée dans la direction opposite de rotation exacte opposant la séquence d'inclination des parties de rampe de la deuxième douille d'entraînement (80) opposant la surface de douille détente correspondent, de manière à fournir un outil de vissage limitant de main gauche ou droite par la rotation des éléments d'accouplement (60, 70, 80) comme un paquet.
